(19) Europäisches Patentamt
European Patent Office
Office européen des brevets

(11) Veröffentlichungsnummer : **0 494 910 B1**

(12) # EUROPÄISCHE PATENTSCHRIFT

(45) Veröffentlichungstag der Patentschrift :
**12.01.94 Patentblatt 94/02**

(51) Int. Cl.$^5$ : **A61K 47/12**

(21) Anmeldenummer : **90914643.3**

(22) Anmeldetag : **03.10.90**

(86) Internationale Anmeldenummer :
**PCT/AT90/00098**

(87) Internationale Veröffentlichungsnummer :
**WO 91/04752 18.04.91 Gazette 91/09**

(54) **AUGENTROPFEN.**

(30) Priorität : **04.10.89 AT 2298/89**

(43) Veröffentlichungstag der Anmeldung :
**22.07.92 Patentblatt 92/30**

(45) Bekanntmachung des Hinweises auf die
Patenterteilung :
**12.01.94 Patentblatt 94/02**

(84) Benannte Vertragsstaaten :
**BE CH DE DK ES FR GB IT LI LU NL SE**

(56) Entgegenhaltungen :
**EP-A- 0 187 433**
**EP-A- 0 288 659**
**WO-A-88/00465**
**Chemical Abstracts, vol. 100, no. 23, 4 June**
**1984, (Columbus, Ohio, US), G. Mingrone et al.,**
**p. 9, abstract 185240c**

(73) Patentinhaber : **EGERER, Ido**
**Niedermarkt 24**
**A-3400 Klosterneuburg (AT)**
Patentinhaber : **MENZEL, Johannes**
**Stallburgstrasse 4/19**
**A-1010 Wien (AT)**

(72) Erfinder : **EGERER, Ido**
**Niedermarkt 24**
**A-3400 Klosterneuburg (AT)**
Erfinder : **MENZEL, Johannes**
**Stallburgstrasse 4/19**
**A-1010 Wien (AT)**

(74) Vertreter : **Pfeifer, Otto, Dipl.-Ing. et al**
**Patentanwälte Schütz und Partner**
**Fleischmanngasse 9**
**A-1040 Wien (AT)**

**Beschreibung**

Die vorliegende Erfindung ist auf dem Gebiete der Augentropfen gelegen und beruht auf der Feststellung, daß durch einen Zusatz von aliphatischen Dicarbonsäuren zu Augentropfen die Verweildauer der Tropfen an der Augenoberfläche verlängert werden kann. Als besonders geeignet hat sich ein Zusatz von Dicarbonsäuren mit 8 bis 11 Kohlenstoffatomen entsprechend der allgemeinen Formel $HOOC-(CH_2)_{6-9}-COOH$ erwiesen, das sind Korksäure, Azelainsäure, Sebacinsäure und Nonandicarbonsäure. Unter diesen Dicarbonsäuren, die zweckmäßig in Form ihrer Natrium- oder Calciumsalze eingesetzt werden, hat sich die Sebacinsäure am geeignetsten erwiesen.In Form einer öligen Schicht bildet sie einen Schutz gegen eine zu rasche Verdunstung der Tränenflüssigkeit am Auge. Ein erfindungsgemäßer Zusatz von beispielsweise Sebacinsäure zu üblichen Augentropfen verlangsamt somit die Verdunstung der eingetropften Flüssigkeit und vermehrt die an der Oberfläche befindliche Ölschicht, welche über die hydrophilen Gruppen der Dicarbonsäure in der wäßrigen Teilschicht des Tränenfilms verankert wird.

Gegenstand der vorliegenden Erfindung ist somit die Verwendung von Dicarbonsäuren mit 8 bis 11 Kohlenstoffatomen als Zusatz bei der Herstellung von Augentropfen. Die Dicarbonsäuren können zweckmäßig in Form ihrer Natrium- oder Calciumsalze eingesetzt werden,am vorteilhaftesten hat sich die Verwendung von Sebacinsäure, insbesondere in Form des Natrium- oder Calciumsalzes, erwiesen.

Aus der Literatur ist die Verwendung von Dicarbonsäuren, insbesondere von Dicarbonsäuren mit 7 - 13 C-Atomen, zur Behandlung verschiedener Hautkrankheiten bekannt. Gemäß der WO-A1-88/00465 werden diese Dicarbonsäuren beispielsweise zur topischen Behandlung der Rosazea verwendet. Die EP-A2-229 654 betrifft die Behandlung entzündlicher Dermatosen, infektiöser Hauterkrankungen und Haarausfall als Folge von Entzündungen oder hormonellen Anomalien. In der US-PS 4 292 326 werden Dicarbonsäuren mit 7 - 13 C-Atomen zur Behandlung von Akne, hyperpigmentierten Dermatosen sowie von Hyperpigmentationen der Haut verwendet. Schließlich wird gemäß der US-PS 4 034 077 Sebacinsäure als Zusatz zu Babysalben und Babypuder in der Konzentration 2 - 30% gegen die Hautirritation beim Tragen von Windeln angewandt.

Diese aus der Literatur bekannten Verwendungsvorschläge von Dicarbonsäuren, insbesondere solcher mit 7 - 13 C-Atomen,beziehen sich ausschließlich auf die Behandlung bei Hautkrankheiten und stellen somit keine Vorwegnahme des Erfindungsgegenstandes dar.

Erfindungsgemäß erzielte Augentropfen mit einem Zusatz an Sebacinsäure weisen besondere Vorteile bei Behandlung von "trockenem Auge" (Keratoconjunctivitis sicca) auf, weil hier eine längere Verweildauer der benetzenden Flüssigkeit an der Augenoberfläche (Hornhaut, Bindehaut) für eine verbesserte Wirksamkeit von entscheidender Bedeutung ist.

Die Sebacinsäure ist eine aliphatische Dicarbonsäure mit der Formel $HOOC(CH_2)_8COOH$, die im Handel erhältlich ist und als absolut reine Lösung in Wasser durch Ionenaustauschchromatographie (HPLC) dargestellt werden kann.

Für die Herstellung der Augentropfen wird zunächst eine Stamnlösung in folgender Weise bereitet:

Eine Suspension von Sebacinsäure und EDTA (Ethylendiamintetraessigsäure) in Wasser wird in einem Ultraschallbad 5 Minuten lang bei Raumtemperatur behandelt; anschließend wird durch langsames Zutropfen von 4n NaOH unter kraftigem Rühren ein pH-Wert von 7,4 eingestellt. In dieser Weise werden eine Endkonzentration an Sebacinsäure von 10% (Gewicht/Volumen) und eine Endkonzentration an EDTA von 0,1% (Gewicht/Volumen) erzielt.

Für die praktische Anwendung sollte eine 0,5%ige Endkonzentration an Sebacinsäure (Verdünnung der Stammlösung 1:20) eingesetzt werden. Zur Bereitung der eigentlichen Augentropfen können als Verdünnungsmedium physiologische Lösungen (z.B. Saline) mit einem pH-Wert von 7,0 bis 7,5 oder Mischungen von gebräuchlichen Augentropfen und physiologischen Lösungen oder auch unverdünnte gebräuchliche Augentropfen dienen.

Die Erfindung wird durch das nachfolgende Beispiel näher veranschaulicht.

AUSFÜHRUNGSBEISPIEL:

Herstellung von Augentropfen zur Behandlung von Keratoconjunctivitis sicca

Aus den folgenden Komponenten werden anwendungsfertige Augentropfen bereitet:

| Sebacinsäure–Stammlösung | 1,0 ml |
|---|---|
| Acidum boricum | 0,3 ml |
| Methylcelluloselösung (0,10 g Methylzellulose, 0,09 g NaCl puriss. Aqua bidest ad 10 ml) | 10,0 ml |
| physiologische NaCl–Lösung | ad 19,0 ml |

Der Zusatz von Sebacinsäurestammlösung zu Augentropfen, welche bei Patienten von Keratoconjunctivitis sicca (trockenes Auge) Verwendung finden, wurde an einem einschlägigen Krankengut erprobt. Von den insgesamt 60 Probanden wurden lediglich von einem einzigen die Augentropfen als nicht angenehm empfunden, die restlichen 59 Probanden zeigten eine sehr gute Verträglichkeit. Mehr als die Hälfte der Patienten wurden über eigenen Wunsch innerhalb eines Beobachtungszeitraumes von 2 Jahren laufend mit den gemäß der erfindundungsgemäßen Verwendung erzielten Augentropfen behandelt.

**Patentansprüche**

1. Verwendung von aliphatischen Dicarbonsäuren der allgemeinen Formel $HOOC\text{-}(CH_2)_{6-9}\text{-}COOH$ bzw. von deren Salzen mit anorganischen Basen als Zusatz zur Herstellung von Augentropfen.

2. Verwendung von Natrium- oder Calciumsalzen der im Anspruch 1 definierten Dicarbonsäuren für den im Anspruch 1 angegebenen Zweck.

3. Verwendung von Sebacinsäure bzw. von deren Natrium- oder Calciumsalzen für den in Anspruch 1 angegebenen Zweck.

**Claims**

1. Use of aliphatic dicarboxylic acids of the general formula $(CH_2)_{6-9}\cdot(COOH)_2$ and their salts with inorganic bases, respectively, as an additive in the production of eye drops.

2. Use of sodium or calcium salts of the dicarboxylic acids defined in claim 1 for the purpose indicated in claim 1.

3. Use of sebacic acid and its sodium or calcium salts, respectively, for the purpose indicated in claim 1.

**Revendications**

1. Utilisation d'acides dicarboxyliques aliphatiques de formule générale $HOOC\text{-}(CH_2)_{6-9}\text{-}COOH$, ou de leurs sels formés avec des bases inorganiques, en tant qu'additifs pour la préparation de gouttes oculaires.

2. Utilisation de sels de sodium ou de calcium des acides dicarboxyliques définis dans la revendication 1, pour l'application indiquée à la revendication 1.

3. Utilisation de l'acide sébacique ou de ses sels de sodium ou de calcium pour l'application indiquée à la revendication 1.